# EUROPÄISCHE PATENTANMELDUNG

(11) **EP 0 636 601 A1**
(43) Veröffentlichungstag der Anmeldung: **01.02.1995**
(21) Anmeldenummer: 94111133.8
(22) Anmeldetag: 18.07.1994
(51) Int. Cl.: C07C 68/00, C07C 69/96

(54) **Verfahren zur Herstellung von Dialkylcarbonaten**

(30) Priorität: 30.07.1993 DE 4325651
(71) Anmelder: BAYER AG, 51368 Leverkusen (DE)
(72) Erfinder: Kricsfalussy, Zoltan, Dr., D-51375 Leverkusen (DE); Waldmann, Helmut, Dr., D-51373 Leverkusen (DE); Traenckner, Hans-Joachim, Dr., D-513075 Leverkusen (DE)

(57) **Zusammenfassung**

C₁-C₄-Dialkylcarbonate können aus den zugrundeliegenden C₁-C₄-Alkanolen, Kohlenmonoxid und Sauerstoff durch Umsetzung dieser Stoffe in Gegenwart von Cu-Salzen erhalten werden, wobei man das Reaktionswasser im Reaktionsgemisch durch mindestens teilweises Ausschleusen auf unter 10 Gew.-% begrenzt.

## Beschreibung

Die Erfindung betrifft ein verbessertes Verfahren zur Herstellung von Dialkylcarbonaten durch Oxycarbonylierung der zugrundeliegenden Alkanole in Gegenwart von Cu-Salzen, wobei man den Gehalt an Wasser auf unter 10 Gew.-% des Reaktionsgemisches begrenzt.

Dialkylcarbonate, insbesondere Dimethylcarbonat, sind Zwischenprodukte mit geringer Toxizität und können giftige Zwischenprodukte, wie Phosgen oder Dimethylsulfat, bei vielen Reaktionen ersetzen. Sie sind ferner nicht korrosiv. Bei ihrer Verwendung entstehen keine umweltschädlichen Nebenprodukte.

Beispiele solcher Reaktionen der Dialkylcarbonate sind die Herstellung von Urethanen aus aliphatischen oder aromatischen Aminen, die wiederum zu den entsprechenden Isocyanaten gespalten werden können. Dimethylcarbonat kann beispielsweise auch Dimethylsulfat bei der Quaternisierung von Aminen oder bei der Methylierung von Phenol oder von Naphtholen ersetzen. Dimethylcarbonat kann ferner dem Fahrbenzin als Oktanzahl-Verbesserer zugesetzt werden, z.B. anstelle von Bleiverbindungen. Angesichts dieser Bedeutung der Dialkylcarbonate fehlt es immer noch an einem technisch einfachen und umweltverträglichen Produktionsverfahren, das für große Kapazitäten ohne wesentliche Nebenproduktbildung oder gekoppelte Stoffkreislaufe geeignet ist.

Zur Herstellung von Dialkylcarbonaten gibt es verschiedene, im kleinen Maßstab bereits auch technisch erprobte Herstellungsverfahren. Die Herstellungswege, die auf der katalytischen Umsetzung von Alkanolen mit Kohlenmonoxid und Sauerstoff gemäß nachstehender Gleichung beruhen, sind von verschiedenen Arbeitsgruppen intensiv bearbeitet worden: 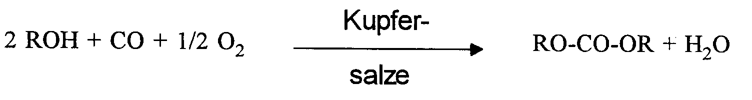
So hat man die als Katalysatoren wirkenden Kupferverbindungen in Form verschiedener Kupfersalze eingesetzt. Bei der Verwendung von Kupfer-II-chlorid als Katalysator gemäß JP-45/11129 (1970) erreicht man unbefriedigende Selektivitäten. Störend ist vor allem die Bildung größerer Mengen an Methylchlorid, das wegen seiner großen Flüchtigkeit dazu neigt, sich ubiquitär in der ganzen Produktionsanlage zu verteilen und praktisch in der gesamten Anlage zu Korrosionen führen kann.

Bessere Selektivitäten erhält man bei der Verwendung von organischen Komplexbildnern (DE-A 21 10 194), aber dort hat man das Problem der Abtrennung der Katalysatorsalze, die im Reaktionsgemisch teils gelöst sind, zum größeren Teil aber als Suspension vorliegen.

Ganz besonders problematisch ist die Durchführung dieser Reaktion gemäß DE-A 27 43 690, da die Katalysatorsalze im Reaktionsgemisch praktisch vollständig ungelöst, sondern lediglich suspendiert sind. Diese Salze müssen durch die Reaktionszone und durch die Kühlaggregate gefördert und nach der Reaktion mechanisch, z.B. mit Hilfe von Zentrifugen, abgetrennt werden. Dies bewirkt neben der bereits erwähnten Korrosion auch Erosionen, schlechten Wärmeübergang sowie Verstopfungen und Verkrustungen.

Um diese Nachteile eines Katalysatorkreislaufs zu vermeiden, hat man Vorschläge gemacht, die Katalysatorsalze im Reaktor suspendiert stationär zu halten und Methanol, CO und Sauerstoff in den Reaktor einzudosieren, während man aus dem Reaktor das gebildete Dialkylcarbonat und das Reaktionswasser zusammen mit im Überschuß eingesetztem Methanol abdestilliert (EP 0 413 215 A2). Dabei besteht das flüssige Reaktionsmedium im wesentlichen aus dem umzusetzenden Alkanol (EP-0 413 215, Seite 3, Zeile 52), so daß das Molverhältnis zwischen Alkanol und Cu-Salz sehr hoch ist (bevorzugt 1:0,01-0,05). Dies hat den Nachteil, daß die Reaktionsgeschwindigkeit relativ niedrig ist. Problematisch dabei ist auch die Notwendigkeit, eine niedrige Dialkylcarbonat-Konzentration einzustellen. Dies ist nicht leicht, da die Reaktion bei hohem Systemdruck durchgeführt wird und die Löslichkeiten von Dialkylcarbonat und auch von Wasser im Reaktionsmedium, das im wesentlichen aus Methanol besteht, sehr hoch sind. Dies bedeutet, daß die Abtrennungen von Dialkylcarbonat und Wasser mit einer relativ großen Inertgas- bzw. Methanolgasmenge erzwungen werden muß.

Im übrigen ist auch bei diesem Vorschlag damit zu rechnen, daß nach einiger Zeit der Katalysator ausgetauscht, bzw. ständig zu einem bestimmten Anteil erneuert werden muß, was mit dem eingangs erwähnten Problem der Katalysatorabtrennung, -regeneration und -rückführung verbunden ist.

Es wurde ein Verfahren zur Herstellung von Dialkylcarbonaten der Formel

(RO)₂CO (I),

worin R für geradkettiges oder verzweigtes Alkyl, bevorzugt für Methyl oder Ethyl, besonders bevorzugt für Methyl steht,
durch Umsetzung der zugrundeliegenden Alkanole der Formel

ROH (II),

worin R die obige Bedeutung hat,
mit Kohlenmonoxid und Sauerstoff in Gegenwart von Cu-Salzen gefunden, das dadurch gekennzeichnet ist, daß die Umsetzung bei 120 bis 300°C, bevorzugt bei 120 bis 180°C und bei 1 bis 70 bar, bevorzugt bei 5 bis 70 bar so durchgeführt wird, daß durch mindestens teilweises Ausschleusen des entstehenden Reaktionswassers der Wassergehalt auf einem Wert von unter 10 Gew.-% des gesamten Reaktionsgemisches gehalten wird.

Auf diese Weise wird der Wassergehalt der Reaktionsmischung kontrolliert und auf niedrige Werte reduziert. Im allgemeinen arbeitet man bei einem Wassergehalt von unter 10 %. Es ist jedoch vorteilhaft, die Wasserkonzentration unter 6 Gew.-% in der Reaktionsmischung zu halten. Besonders günstig für hohen Umsatz bei gleichzeitig sehr hohen Selektivitäten ist es, die Reaktion bei Werten von unter 3 Gew.-% zu fahren.

Bevorzugt wird das Verfahren der Erfindung bei Verwendung einer Cu-Salz enthaltenden Salzschmelze, wobei als Cu-Salz Cu-I- und Cu-II-Verbindungen sowie Gemische davon in Frage kommen. Grundsätzlich sind alle bekannten Cu-Salze, sofern sie in der Salzschmelze nur einigermaßen löslich sind, geeignet.

Geeignet sind neben den Halogeniden, wie z.B. den Chloriden oder den Bromiden, auch die Cyanide, Rhodanide, Sulfate, Nitrate, Carbonate, Acetate, Formiate, Oxalate, Alkoholate, z.B. Cu-methoxychlorid. Cu kann auch in Form komplexierter Verbindungen, wie den Acetylacetonaten, oder Cu-N-komplexen, wie Cu-Pyridin- oder auch Cu-Dipyridylkomplexen, eingesetzt werden.

Für die Salzschmelze nimmt man im allgemeinen Gemische von Salzen, die einen niedrigen Schmelzpunkt haben, d.h., die ein Eutektikum bilden. Es ist daher vorteilhaft, Mengenverhältnisse der Salze entsprechend der Zusammensetzung des Eutektikums zu verwenden. Solche Eutektika können von Cu-Salzen untereinander oder von Cu-Salzen und weiteren Salzen gebildet werden.

Neben den Cu-Salzen kann man daher im Prinzip alle chemisch inerten, oder auch im erfindungsgemäßen Sinne katalytisch wirksamen, d.h., die Aktivierungsenergie für die Oxycarbonylierung von Alkanolen herabsetzenden Salze verwenden. Neben Cu-Salzen kann man dabei eine breite Anzahl von Salzen oder salzartigen Verbindungen verwenden. In der Regel setzt man Gemische von Cu-Salzen und solchen salzartigen Verbindungen ein. Bevorzugt setzt man die Halogenide der 1. bis 3. Haupt- und Nebengruppen ein. Besonders geeignet sind die Chloride der Alkalimetalle, wie NaCl oder KCl, oder Chloride der Erdalkalien, wie CaCl₂ oder MgCl₂, sowie ZnCl₂. Aber auch die Verwendung von weniger häufigen Verbindungen, wie die Chloride von Thallium, Indium oder Gallium, ist möglich.

Eine gut geeignete Schmelze besteht beispielsweise aus Cu-I-Chlorid und KCl in unterschiedlichen Mengenverhältnissen. Im allgemeinen wählt man Mischungen mit einem hohen Gehalt an Cu-Verbindungen z.B. ein Gewichtsverhältnis von Cu-I-Chlorid zu KCl von 60 bis 75 zu 40 bis 25.

Die Reaktionstemperatur beträgt im allgemeinen etwa 120°C bis 300°C, bevorzugt 120°C bis 180°C, typische Reaktionstemperaturen liegen bei 120°C bis 150°C.

Die Reaktion kann bei Normaldruck durchgeführt werden. Um eine genügend hohe Reaktionsgeschwindigkeit zu erhalten, ist es jedoch zweckmäßig, bei höherem Druck, z.B. bei 5 bis 70 bar, bevorzugt bei 10 bis 50 bar, besonders bevorzugt bei 25 bis 50 bar, zu arbeiten.

Die Molverhältnisse der zum Einsatz gebrachten Reaktionskomponenten sind für die Reaktionsgeschwindigkeit und für die Selektivität der Reaktion von Bedeutung. Als Nebenprodukte treten bei Nichtbeachtung dieser Zusammenhänge beispielsweise das Dimethylacetal des Formaldehydes oder - was besonders störend ist - Methylchlorid auf.

Im allgemeinen wählt man einen molaren Überschuß von Methanol zu Kohlenmonoxid und wiederum einen Überschuß von Kohlenmonoxid zu Sauerstoff, höchstens jedoch molare Mengen von CO bzw. O₂. So wählt man Molverhältnisse von Alkanol zu CO und O₂ von 1:1-0,01:1-0,01, bevorzugt von 1:0,5-0,02:0,3-0,02. Dabei resultieren ein Methanolumsatz von beispielsweise 10 bis 50 % und ein CO-Umsatz von 10 bis 80 %. Sauerstoff wird im allgemeinen völlig umgesetzt. Bei der Mengendosierung müssen selbstverständlich die Explosionsgrenzen beachtet werden. Gegebenenfalls kann in Gegenwart von Inertgasen, wie N₂ oder CO₂, gearbeitet werden.

Der Sauerstoff kann beispielsweise in Form von atmosphärischer Luft oder von mit O₂ angereicherter Luft eingesetzt werden.

Die nicht umgesetzten Anteile von Methanol und CO können nach Abtrennung von Dialkylcarbonat und H₂O, gegebenenfalls auch von CO₂, zurückgeführt werden.

Zur Ausschleusung des Wassers können alle bekannten verfahrenstechnischen Maßnahmen herangezogen werden. Man kann beispielsweise so verfahren, daß man nach einem bestimmten Reaktionsfortschritt, z.B. bei einem Umsatz des Alkanols von 8 bis 35 %, bevorzugt 10 bis 27 %, besonders bevorzugt 15 bis 25 %, die organischen Anteile des Reaktionsgemisches in an sich bekannter Weise abtrennt und anschließend oder gleichzeitig das Reaktionswasser z.B. durch Destillation abtrennt. Eine solche Destillation erfolgt beispielsweise im Falle der Herstellung von Dimethylcarbonat in der Form, daß man ein Reaktionsgemisch mit einer Zusammensetzung von 20 bis 25 % Dimethylcarbonat, 4 bis 6 % Wasser, Rest Methanol in die Mitte einer Fraktionierkolonne mit ca. 25 theoretischen Böden leitet und bei etwa atmosphärischem Druck als Sumpfprodukt Wasser ausschleust, wobei als Kopfprodukt Gemische von 20 bis 27 % Dimethylcarbonat und Rest Methanol erhalten werden. Dieses Kopfprodukt kann erneut in die Reaktion mit Kohlenmonoxid und einem Sauerstoff enthaltenden Gas eingesetzt werden.

Bei dieser erneuten Umsetzung des Reaktionsgemisches kann man beispielsweise die Umsetzung bis zu einem Wassergehalt von 4 bis 6 % vorantreiben, wobei man bei sehr hohen Selektivitäten Reaktionsgemische mit einem Dialkylcarbonatgehalt von 50 bis 55 Gew.-% erhält. Durch erneute und gegebenenfalls wiederholte Wasserausschleusung kann man bis zu einem Dialkylcarbonatgehalt von etwa 75 % gelangen.

Der besondere Vorteil des erfindungsgemäßen Verfahrens liegt darin, daß man Reaktionsgemische mit hohen Dialkylcarbonatgehalten, beispielsweise 50 bis 70 Gew.-% erhält, die die Isolierung des reinen Dialkylcarbonates in besonders einfacher und wirtschaftlicher Weise gestatten.

Als verfahrenstechnische Methoden zur Entfernung des Reaktionswassers kommen auch weitere Entwässerungsverfahren, z.B. Azeotropdestillation, gegebenenfalls unter Zugabe eines Azeotropbildners zum Reaktionsgemisch in Frage. Ferner sind auch Extraktionsmethoden in vorteilhafter Weise anzuwenden. Es ist aber auch möglich, das Reaktionswasser chemisch oder adsorptiv zu binden. Die destillative Entfernung ist jedoch bevorzugt.

Das erfindungsgemäße Verfahren kann in den verschiedenen bekannten Reaktortypen durchgeführt werden. Man kann beispielsweise in einem Rührwerkskessel mit Begasungsrührer arbeiten. Man kann diskontinuierlich, aber auch kontinuierlich arbeiten. Für die kontinuierliche Durchführung eignen sich auch Reaktionskessel-Kaskaden mit beispielsweise 3 bis 5 Reaktoren. Für das Verfahren ist aber auch eine ein- oder mehrstufige Blasensäule geeignet.

Die Gasbelastung kann in weiten Grenzen variiert werden, je nach Druck und Temperatur, so daß Raum-Zeit-Ausbeuten zwischen 10 und 200 g/l.h erreicht werden können.

Die Reaktionswärme kann durch Kühlaggregate entfernt werden. In einer besonderen Durchführungsform arbeitet man jedoch in einem sogenannten Siedereaktor, bei dem die Reaktionswärme durch verdampfendes Produkt abgeführt wird. So wird beispielsweise bei Flüssigeinspeisung von Alkanol die Reaktionswärme durch das Verdampfen des Alkanols aufgenommen; Kühlaggregate am Reaktor sind dabei nicht erforderlich. Der Austrag der Reaktionsprodukte Dialkylcarbonat und Wasser aus dem Reaktor erfolgt hierbei durch den Gasstrom. Die Konzentration der Stoffe im Gasstrom ist vom Druck und Temperatur abhängig. Es kann daher, insbesondere bei höherem Druck, vorteilhaft sein, kurzzeitig zu entspannen und abschließend wieder unter Druck zu arbeiten (sog. Pressure Swing-Technik). So kann man beispielsweise bei der Herstellung von Dimethylcarbonat so verfahren, daß man 1 min bis 10 min bei höherem Druck, beispielsweise bei 25 bis 50 bar, arbeitet und anschließend den Systemdruck auf etwa 10 bis 0,8 bar, bevorzugt 3 bis 1 bar reduziert. Dabei bleibt die Schmelze vollständig in der Reaktionszone und die Organika, z.B. Dimethylcarbonat und Methanol, destillieren praktisch vollständig aus dem Reaktor aus. Diese taktweise Änderung des Systemdruckes, die die Abtrennung der Reaktionsprodukte erleichtert, ist für das erfindungsgemäße Verfahren besonders geeignet.

Als Werkstoffe für die Reaktoren eignen sich beispielsweise korrosionsbeständige Edelstähle, Stahl-Emaille, Glas oder Sondermetalle wie Tantal.

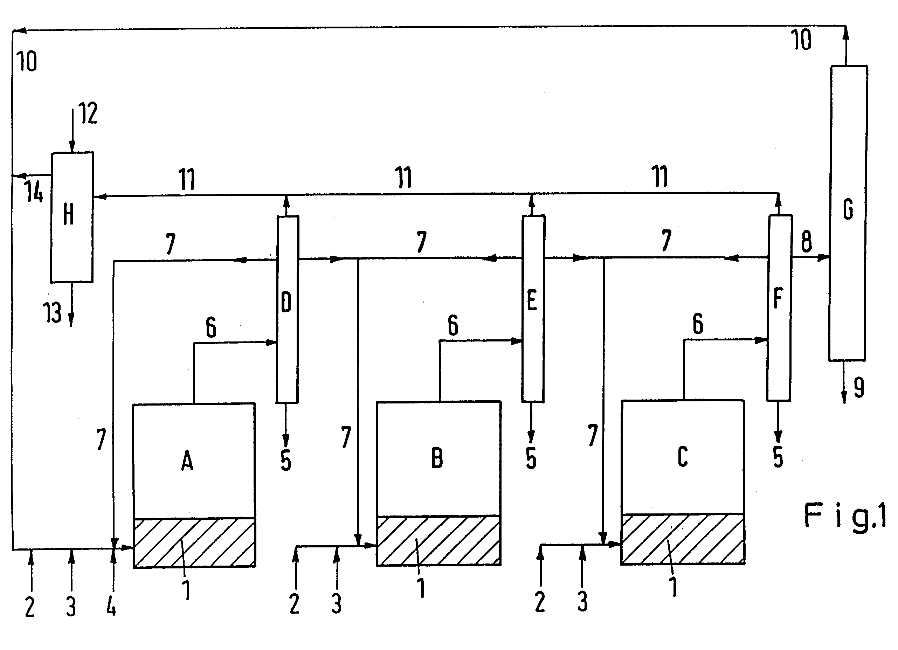

Das erfindungsgemäße Verfahren kann auch technisch im größeren Maßstab durchgeführt werden. Dabei kann man beispielsweise gemäß Fig. 1 in einer mehrstufigen, z.B. 3-stufigen Reaktionskaskade, wobei auf jede Reaktoreinheit eine Destillationskolonne zur Wasserausschleusung angebracht ist, einen Produktstrom mit 55 bis 57 Gew.-% Dialkylcarbonat erhalten.

In Fig. 1 sind folgende Apparate dargestellt: Drei Reaktoren A, B und C; drei zugehörige Destillationskolonnen D, E und F zur Wasserausschleusung; die Destillationskolonne G zur Gewinnung von Dialkylcarbonat; ein Gaswäscher H zum Auswaschen von CO₂ (gebildet als unerwünschtes Nebenprodukt durch Oxidation von CO). In Fig. 1 sind weiterhin folgende Reaktionsteilnehmer dargestellt: Cu-Salz enthaltende Schmelze (1) in A, B und C; zugeführtes CO (2) bzw. O₂ (3); zugeführtes Alkanol (4); ausgeschleustes H₂O (5); entnommenes organisches Reaktionsgemisch (6); zurückzuführendes Gemisch Alkylcarbonat/Alkanol (7); Gemisch Alkylcarbonat/Alkanol (8) zur Gewinnung von Alkylcarbonat; aufkonzentriertes Alkylcarbonat (9); zurückzuführendes Alkanol (10), gegebenenfalls im Gemisch mit Alkylcarbonat; aus D, E und F abströmendes CO/CO₂-Gemisch (11), das gegebenenfalls resiliches O₂ enthalten kann; zulaufende Natronlauge (12) für die Gaswäsche; ablaufendes NaHCO₃ (13); zurückzuführendes CO (14), gegebenenfalls zusammen mit restlichem O₂. Auf Inertgas wurde der Einfachheit halber nicht hingewiesen.

### Beispiel 1

In einem mit Ta-Einsatz versehenen Stahlbehälter, ausgestattet mit Einleitungsrohr, Temperaturmessung und Druckhaltung, legte man 94 ml eines bei 150°C geschmolzenen Salzgemisches von 72 Gew.-% Cu-I-chlorid und 28 Gew.-% KCl vor. Dazu dosierte man 390 g/h Methanol, 36 l/h CO und 42 l/h Luft bei 150°C und 50 bar. Nach Entspannung dosierte man die Reaktionsmischung unaufgearbeitet wieder in den Reaktor.
Nach dreimaliger Rückführung betrug der Methanol-Umsatz 27 % und die Dimethylcarbonat-Selektivität 98 %.

### Beispiel 2

Das nach dreimaliger Rückführung erhaltene Reaktionsgemisch aus Beispiel 1 entwässerte man in einer Destillationskolonne und dosierte in den Reaktor unter Bedingungen wie in Beispiel 1 beschrieben.
Nach dreimaliger Rückführung betrug der Methanol-Umsatz 55 % und die Dimethylcarbonat-Selektivität 97 %.

### Beispiel 3

Eine Mischung aus 50 % Methanol und 50 % Dimethylcarbonat dosierte man unter Bedingungen wie in Beispiel 1 beschrieben in den Reaktor.
Nach einmaligem Durchsatz betrug der Methanol-Umsatz 56 % und die Dimethylcarbonat-Selektivität 98 %.

### Beispiel 4

Eine Mischung aus 40 % Methanol und 60 % Dimethylcarbonat dosierte man unter Bedingungen wie in Beispiel 1 beschrieben in den Reaktor.
Nach einmaligem Durchsatz betrug der Methanol-Umsatz 65 % und die Dimethylcarbonat-Selektivität 98 %.

### Beispiel 5

Eine Mischung aus 30 % Methanol und 70 % Dimethylcarbonat dosierte man unter Bedingungen wie in Beispiel 1 beschrieben in den Reaktor.
Nach einmaligem Durchsatz betrug der Methanol-Umsatz 73 % und die Dimethylcarbonat-Selektivität 98 %.

## Patentansprüche

1. Verfahren zur Herstellung von Dialkylcarbonaten der Formel
(RO)₂CO,
worin R für geradkettiges oder verzweigtes Alkyl, bevorzugt für Methyl oder Ethyl, besonders bevorzugt für Methyl steht,
durch Umsetzung der zugrundeliegenden Alkanole der Formel
ROH,
worin R die obige Bedeutung hat,
mit Kohlenmonoxid und Sauerstoff in Gegenwart von Cu-Salzen, dadurch gekennzeichnet, daß die Umsetzung bei 120 bis 300°C, bevorzugt bei 120 bis 180°C und bei 1 bis 70 bar, bevorzugt bei 5 bis 70 bar so durchgeführt wird, daß durch mindestens teilweises Ausschleusen des entstehenden Reaktionswassers der Wassergehalt auf einen Wert von unter 10 Gew.-% des gesamten Reaktionsgemisches gehalten wird.

2. Verfahren nach Anspruch 1, dadurch gekennzeichnet, daß der Wassergehalt auf einen Wert von unter 6 Gew.-%, bevorzugt von unter 3 Gew.-% gehalten wird.

3. Verfahren nach Anspruch 1, dadurch gekennzeichnet, daß das Cu-Salz in Form einer ein Cu-Salz enthaltenden Salzschmelze eingesetzt wird.

4. Verfahren nach Anspruch 3, dadurch gekennzeichnet, daß eine Schmelze von Cu-I-chlorid und KCl eingesetzt wird.

5. Verfahren nach Anspruch 4, dadurch gekennzeichnet, daß ein Gewichtsverhältnis CuCl : KCl = 60 bis 75 : 40 bis 25 eingestellt wird.

6. Verfahren nach Anspruch 1, dadurch gekennzeichnet, daß ein Molverhältnis von Alkanol:CO:O₂ = 1:1 - 0,01:1 - 0,01, bevorzugt 1:0,5 - 0,02:0,3 - 0,02 eingestellt wird.

7. Verfahren nach Anspruch 1, dadurch gekennzeichnet, daß man die Ausschleusung des Reaktionswassers destillativ durchführt.

8. Verfahren nach Anspruch 7, dadurch gekennzeichnet, daß man das Reaktionswasser gemeinsam mit gebildetem Dialkylcarbonat und dem zugrundeliegenden Alkanol aus dem Reaktionsgemisch destilliert, das dabei erhaltene Destillat in eine Fraktionierkolonne leitet, Wasser als Sumpfprodukt erhält und ausschleust und Dialkylcarbonat und Alkanol als Kopfprodukt in die Reaktion zurückführt.

9. Verfahren nach Anspruch 1, dadurch gekennzeichnet, daß man kontinuierlich in einer Rührwerkskessel-Kaskade mit 3 bis 5 Reaktoren arbeitet.

10. Verfahren nach Anspruch 1, dadurch gekennzeichnet, daß man zur Herstellung von Dimethylcarbonat die Reaktion bei 25 bis 50 bar durchführt und absatzweise auf 10 bis 0,8 bar, bevorzugt 3 bis 1 bar zum Ausdestillieren der organischen Anteile des Reaktionsmediums entspannt und anschließend den Druck wieder auf 25 bis 50 bar ansteigen laßt.
